# EUROPEAN PATENT SPECIFICATION

(11) **EP 2 244 625 B1**
(45) Date of publication and mention of the grant of the patent: **04.04.2018**
(21) Application number: 09708195.4
(22) Date of filing: 04.02.2009
(51) Int. Cl.: A61B 1/018, A61B 1/00, A61B 8/08, A61B 8/12

(54) **ADAPTOR FOR ENDOSCOPIC ORIENTATION OF AN ELONGATE MEDICAL DEVICE**
ADAPTER FÜR ENDOSKOPISCHE AUSRICHTUNG EINER LÄNGLICHEN MEDIZINISCHEN VORRICHTUNG
ADAPTATEUR POUR UNE ORIENTATION ENDOSCOPIQUE D'UN DISPOSITIF MÉDICAL ALLONGÉ

(30) Priority: 05.02.2008 US 26391 P
(43) Date of publication of application: 03.11.2010
(73) Proprietor: Cook Medical Technologies LLC, Bloomington, IN 47404 (US)
(72) Inventor: MCGRATH, Darach, County Tipperary (IE)
(74) Representative: Garratt, Peter Douglas
(86) International application number: PCT/US2009/033033
(87) International publication number: WO 2009/100106

(56) References cited:
- WO-A-2006/094044
- US-A1- 2005 033 319
- US-A1- 2007 270 640

## Description

### RELATED APPLICATIONS

This application claims the benefit under 35 U.S. C. § 119(e) of U.S. Provisional Application Serial No. 61/026,391, filed February 5, 2008, which is incorporated herein by reference in its entirety.

### TECHNICAL FIELD

This invention generally relates to an adaptor for elongate medical devices that are insertable into an endoscope, and in particular to an adaptor for orienting the medical device relative to the endoscope.

### BACKGROUND

Endoscopic devices and procedures may be used to diagnose, monitor and treat various conditions by close examination of the internal organs. By way of background, a conventional endoscope generally is an instrument having a device for visualizing the interior of an internal region of a body and a lumen for inserting one or more treatment devices therethrough. A wide range of applications have been developed for the general field of endoscopes including by way of example the following: arthroscope, angioscope, bronchoscope, choledochoscope, colonoscope, cytoscope, duodenoscope, enteroscope, esophagogastro-duodenoscope (gastroscope), laparoscope, laryngoscope, nasopharyngo-neproscope, sigmoidoscope, thoracoscope, and utererscope (individually and collectively, "endoscope").

In some endoscopic devices, visualization of the internal regions may be obtained using a video camera. The video camera provides a viewing field to observe the surgical instrumentation or procedure within the viewing field. Medical ultrasound has also been used to monitor a surgical procedure within a viewing field. Endoscopic ultrasound (EUS) utilizes high frequency sound waves to create an image of living tissue or an echogenic surface. Ultrasound waves are emitted from transducers located at the distal end of an endoscope. Surgical instruments having an echogenic surface reflect the ultrasound waves and enable an endoscopist to monitor the location of the device within the patient.

In some procedures, medical devices are inserted through the endoscope to access the internal organs. For example, an elongate device, such as a needle, may be inserted through an accessory channel of the endoscope for removing tissue or cell samples, or injecting a medication or diagnostic fluid. Fine needle aspiration (FNA) has been a well accepted method for obtaining tissue samples for pathologic or histological analysis in diagnosing a lesion, tumor neoplasm or other abnormality in internal organs. EUS and EUS-guided fine needle aspiration (EUS-FNA) have become important tools in the evaluation of tissue and cell abnormalities.

Surgical techniques for obtaining tissue samples accessible through an endoscopic device such as an ultrasound-endoscope using a fine needle usually require repeated needle sampling at a tissue site to ensure an adequate sample for analysis. Typically in a biopsy procedure, a distal tip of the elongate medical device is extended distally from a port of the endoscope channel to reach the sample site. A sample is removed from the patient through the distal tip, the distal tip is retracted back through the channel and the sample is collected. The elongate medical device is reinserted into the channel of the endoscope and the distal tip of the device is subsequently re-extended distally through the endoscope to collect another sample. When an EUS system is used, it is important for the endoscopist to be able to re-extend the distal tip of the medical device at the correct angle so that the tip is visible in the EUS plane where the ultasound waves are emitted. With the tip extended in the EUS plane, a subsequent sample may be obtained.

One problem with repeated sampling using an elongate device viewed with an EUS system, such as a needle, is that the distal tip of the device may curve or bend upon exiting a port of the endoscope. The deformation of the device may be such that the bend or curve formed during the previous extension remains in the distal tip when the distal tip is distally re-extended from the endoscope for the subsequent sampling procedure. The curved distal end of the sampling device will impair the endoscopist's ability to view the distal tip if the bend causes the tip of the device to project at an angle that is out of the EUS viewing plane. Simply reinserting the elongate device through the accessory channel and distally extending the tip may not allow the endoscopist to view the re-extended tip in the EUS viewing plane once the bend or curve has been introduced into the distal tip of the medical device. Similarly, other devices having an orientatable distal end extending from an endoscope may need to be oriented upon reinsertion through a channel of the endoscope so that the distal end of the medical device extends in the desired viewing plane. Viewing devices such as an imaging camera may also have requirements for a medical device to be re-extended into the viewing plane.

Document US 2007/0270640 discloses an endoscope tool coupling that facilitates the placement and positioning of a surgical tool within an endoscope. The tool coupling may be configured to accommodate differences in lengths between the surgical tool and the endoscope, which may allow a user to precisely position and maintain the distal end of a surgical tool in a desired location relative to the distal end of the endoscope despite differences and variations in lengths. The tool coupling may have an adjustable configuration to accommodate variations in tool and/or endoscope lengths. A locking arrangement may be provided to secure the coupling in a selected configuration for maintaining the surgical tool in a desired location relative to the endoscope. The tool coupling may be configured to allow for the withdrawal and reintroduction of the same or a similar surgical tool without changing or impacting the original length adjustment and/or preload for the particular tool/endoscope arrangement.

For the foregoing reasons, it is desirable to have an adaptor for an endoscope, as taught herein, that orients the distal tip of an elongate medical device that extends distally from the endoscope relative to the endoscope.

### SUMMARY OF THE INVENTION

Accordingly, it is an object of the present invention to provide an adaptor for an endoscope having features that resolve or improve on one or more of the above-described drawbacks. The scope of the present invention is set forth in the appended claims.

The foregoing object is obtained by providing an adaptor to orient an elongate medical device in relation to an endoscope. The adaptor includes a first portion having a distal end connectable and rotationally securable to an endoscope and a proximal end. The first portion further includes a first lumen defined longitudinally therethrough and one of an orienting key or a keyway extending longitudinally at least partially along the first portion. The adaptor further includes a second portion connectable to the first portion and having a second lumen extending longitudinally through the second portion and operably connectable to the first lumen. The second portion has the other of the key or the keyway extending longitudinally at least partially along the second portion. The keyway is configured to releasably mate with the key to orient and rotationally secure the second portion relative to the first portion. The second portion is configured to receive an elongate medical device longitudinally movable in relation to the second portion and rotationally secured relative to the second portion, the elongate medical device adapted to extend distally through the endoscope and having a tip that is orientable in relation to a distal portion of the endoscope portion. The adaptor is configured to orient the tip portion relative to the endoscope.

In another aspect, an system is provided to orient an elongate medical device relative to an endoscope. The system includes an endoscope, an adaptor connected to the endoscope and an elongate medical device rotationally secured to a second portion of the adaptor. The adaptor includes a first portion and a second portion. The first portion includes a connecting portion to rotationally secure a distal end of the first portion to the endoscope, a first lumen extending longitudinally through the first portion and operably connectable to a working channel of the endoscope, and one of an orienting key or a keyway extending longitudinally at least partially along the first portion. The second portion is releasably connectable to the first portion and includes the other of the key or the keyway extending longitudinally along at least a portion of the second portion and a second lumen operably connectable to the first lumen and configured to receive an elongate medical device rotationally secured in relation to the second portion therethrough. The other of the key or the keyway is configured to engage the key or the keyway on the first portion to rotationally fix the second portion in relation to the first portion.

In another aspect, a method of orienting an elongate medical device extending through a working channel of an endoscope and having a distal tip portion extending distally through the endoscope. The method employs an adaptor including a first portion and a second portion, the second portion having an elongate medical device rotationally secured thereto. The method includes connecting a first portion of the adaptor to the endoscope so as to rotationally secure the first portion in relation to the endoscope and operably connect a first lumen of the first portion with the working channel of the endoscope. The method further includes extending a distal portion of an elongate medical device longitudinally through the first lumen and into the working channel of the endoscope and connecting the second portion to the first portion and rotationally securing the second portion to the first portion by engaging one of a key or a keyway on the second portion to the other of the key or the key way on the first portion.

Advantages of the present invention will become more apparent to those skilled in the art from the following description of the preferred embodiments of the invention which have been shown and described by way of illustration. As will be realized, the invention is capable of other and different embodiments, and its details are capable of modification in various respects. Accordingly, the drawings and description are to be regarded as illustrative in nature and not as restrictive.

### BRIEF DESCRIPTION OF THE DRAWINGS

FIG. 1 is a side perspective view of an adaptor according to the present invention:
FIG. 2 is a side perspective view of the adaptor shown in FIG. 1 with a first portion of the adaptor connected to an endoscope;
FIG. 3A is a cross-sectional view of an alternative key/keyway configuration;
FIG. 3B is a cross-sectional view of another alternative key/keyway configuration;
FIG. 3C is a cross-sectional view of another alternative key/keyway configuration;
FIG. 3D is a cross-sectional view of another alternative key/keyway configuration;
FIG. 3E is a cross-sectional view of another alternative key/keyway configuration;
FIG. 4 is a side view of a portion of an elongate medical device including a sheath and a needle;
FIGS. 5A and 5c illustrate an exemplary handle of a needle device that may be oriented with the adaptor;
FIG. 5B is a cross-sectional view through the second portion shown in FIG. 5A;
FIG.6 side view of an exemplary endoscope having an accessory channel for attachment of the adaptor of shown in FIG. 1;
FIG. 7A is a partial view of an elongate medical device extending distally from an endoscope into a viewing field;
FIG. 7B is a partial view of the elongate medical device shown in FIG. 7A re-extending distally from an endoscope and curving out of the viewing field; and
FIG. 7C is a partial view of the elongate medical device shown in FIG. 7A re-extending distally from an endoscope into the viewing field using the adaptor shown in FIG. 1.

### DETAILED DESCRIPTION OF THE EMBODIMENTS

The invention is described with reference to the drawings in which like elements are referred to by like numerals. The relationship and functioning of the various elements of this invention are better understood by the following detailed description. However, the embodiments of this invention are not limited to the embodiments illustrated in the drawings. It should be understood that the drawings are not to scale, and in certain instances details have been omitted which are not necessary for an understanding of the present invention, such as conventional fabrication and assembly.

As used in the specification, the terms proximal and distal should be understood as being in the terms of a physician operating an endoscope and an elongate medical device for insertion into a patient. Hence the term distal means the portion of the device that is farthest from the physician and the term proximal means the portion of the device that is nearest to the physician.

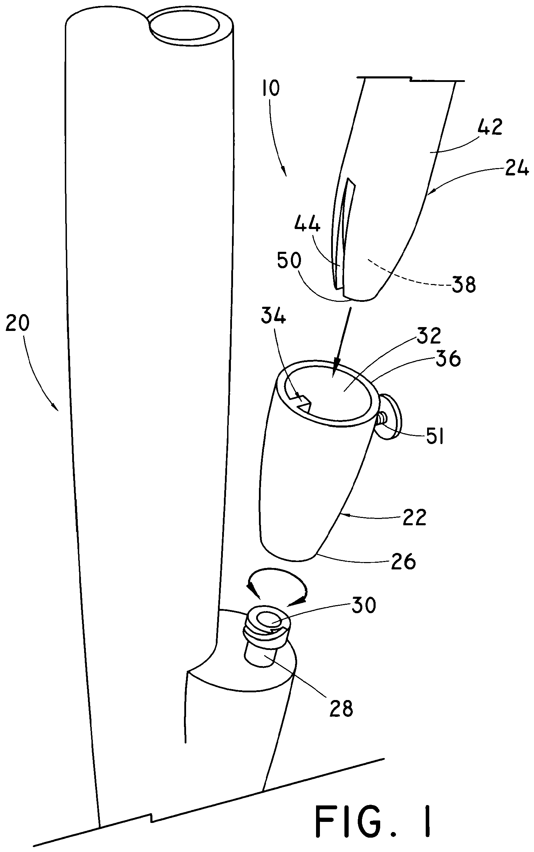

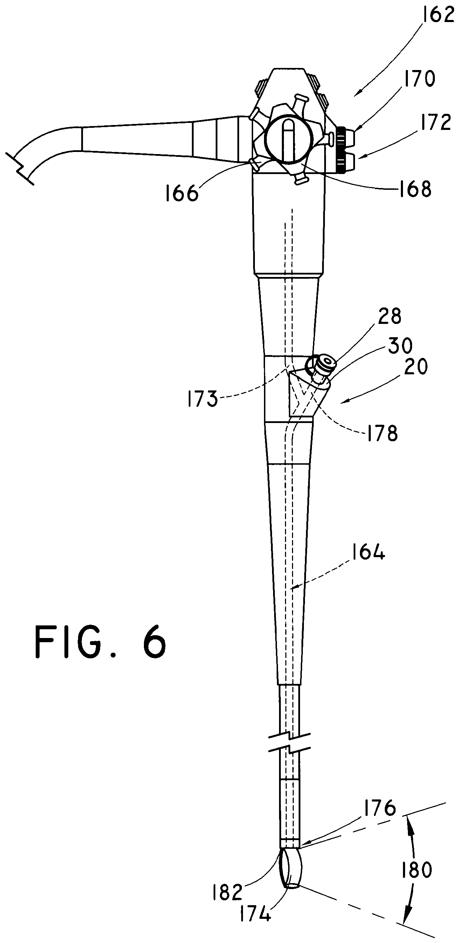

FIG. 1 illustrates an adaptor 10 in accordance with embodiments of the present invention. The adaptor 10 is configured for removable connection to an endoscope 20 (shown in FIG. 6 and described in more detail below). The adaptor 10 includes a first portion 22 and a second portion 24. The first portion 22 and the second portion 24 may be releasably connectable to each other wherein the first portion 22 includes one of a key or a keyway and the second portion 24 includes the other of the key or the keyway to rotationally secure the second portion 24 in relation to the first portion 22. FIG. 1 illustrates the adaptor 10 having one of the many combinations possible for the key and the keyway.

As shown in FIG. 1, the first portion 22 includes a distal end 26 that may be connected to a connector 28 on an accessory channel 30 of the endoscope 20. The first portion 22 further includes a lumen 32 extending longitudinally therethrough. An orienting key 34 is shown in FIG. 1 extending into the lumen 32 at a proximal end 36 of the first portion 22. The key 34 extends longitudinally along at least a portion of the lumen 32 and is not required to extend all the way to the proximal end 36. The first portion 22 may be connected to the connector 28 of the endoscope 20 using any connection known to one skilled in the art that will secure the rotational orientation of the first portion 22 once the first portion 22 is connected to the endoscope 20. Non-limiting exemplary connections include threaded (as shown), snap-fit, and the like. The first portion 22 may be connected and secured to the endoscope 20 in any rotational orientation in relation to a longitudinal axis of the endoscope 20. Once the first portion 22 is connected to the endoscope 20, the first portion 22 remains secured in position on the endoscope throughout the procedure. The first portion 22 may be removed from the endoscope 20 once the procedure has been completed.

The second portion 24 of the adaptor 10 includes a lumen 38 extending longitudinally therethough. The second portion 24 is configured to be removably connected to the first portion 22. When the first portion 22 is connected with the second portion 24, the lumens 32 and 38 are operably connected. The second portion 24 further includes an exterior surface 42 having a keyway 44 for engaging the key 34 of the first portion 22. As shown in FIG. 1, the second portion 24 is sized and shaped so that at least a distal end 50 of the second portion 24 is received into the lumen 32 at the proximal end 36 of the first portion 22. The keyway 44 of the second portion 24 engages the key 34 of the first portion 22 so that the second portion 24 is rotationally secured in relation to the first portion 22 and also to the endoscope 20. One skilled in the art will understand that the second portion 24 may be releasably connected to the first portion 22 by having the distal end 50 of the second portion 24 received over the proximal end 36 of the first portion 22. In one alternative embodiment, the key or keyway may be on the exterior of the first portion 22 and the other of the key or keyway may extend into the lumen 38 of the second portion 24. In other words, the second portion 24 may be configured to slide over the first portion 22.

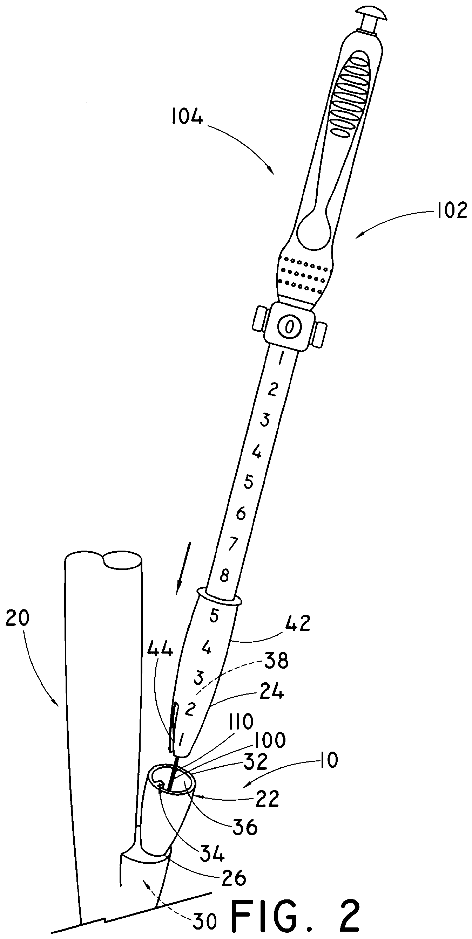

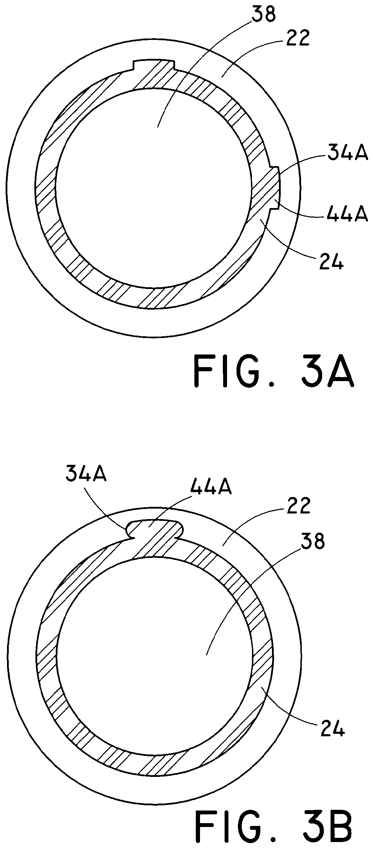

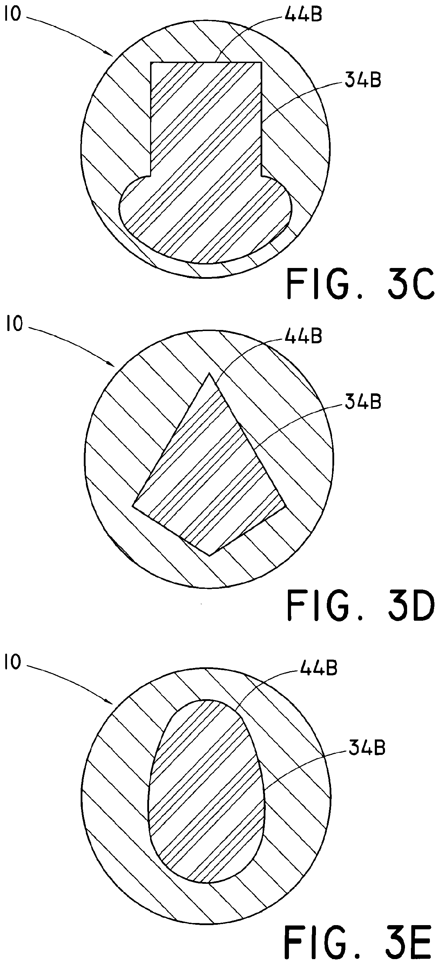

The key and keyway configuration described above for the adaptor 10 may have any size and shape known to one skilled in the art. By way of non-limiting example, the shape may be rectangular, circular, oval, triangular, and the like. Any releasably mating configuration may be used to rotationally secure the second portion with respect to the first portion of the adaptors described herein. Two or more key/keyway pairs may be used to orient the first and second portions and rotationally secure the second portion with respect to the first portion. Additional example of exemplary key and keyway configurations are shown in FIG. 3A and 3B. Key 34a and keyway 44a refer to alternative configurations from the key 34 and keyway 44 shown in FIGS. 1 and 2 where key 34a is on the second portion 24 and keyway 44a is on the first portion 22. The key and keyway may also be formed from the shape of the first portion and the second portion themselves. FIGS. 3C-3E illustrate a key/keyway configuration where the shape of the exterior of one portion forms the key 34b and the interior of the other portion forms the keyway 44b. In each of the embodiments shown in FIGS. 3C-3E, the key 34b/keyway 44b orient and rotationally secure the second portion in relation to the first portion. The exterior of the adaptor may be cylindrical and include a lumen that extends therethrough as described above.

The connection between the first portion 22 and the second portion 24 may be friction fit so that the second portion 24 remains connected to the first portion 22 until the physician disengages the first portion 22 from the second portion 24. The first portion 22 may also include a releasable locking mechanism, such as a thumb screw 51, to hold the second portion 24 in a longitudinally secured position during a sampling procedure. The thumb screw 51 may also be used to limit or adjust the axial position of the second portion relative to the first portion. The connection between the first portion 22 and the second portion 24 is configured to be repeatedly removed and reconnected to reinsert the second portion 24 in an oriented direction in relation to the first portion 22 and the endoscope 20. One skilled in the art will understand that other connections between the first portion 22 and the second portion 24 are possible.

The second portion 24 is configured to receive an elongate medical device therethrough as will be described in more detail below. The elongate medical device may be longitudinally movable within the second portion 24, but not radially rotatable in relation to the second portion 24.

FIG. 2 illustrates the first portion 22 of the adaptor 10 connected to the accessory channel 30 of the endoscope 20. Once the first portion 22 is connected to the endoscope 20, the key 34 remains secured in position relative to the endoscope 20 until the procedure is completed. The initial rotational position of the key 34 in relation to the endoscope does not matter as long as the key 34 remains in the same position in relation to the endoscope throughout a procedure where orientation of the medical device is important. The second portion 24 is shown being advanced toward the first portion 22 in FIG. 2. A portion of an elongate medical device 100 is shown extending from the second portion 24 and into the first portion 22 to enter the endoscope 20 through the accessory channel 30 to a channel of the endoscope (not shown). The keyway 44 of the second portion 24 may be aligned with the key 34 of the first portion 22 to rotationally secure the second portion 24 in relation to the first portion 22. The second portion 24 and the medical device 100 may be repeatedly removed from and reinserted into the first portion 22.

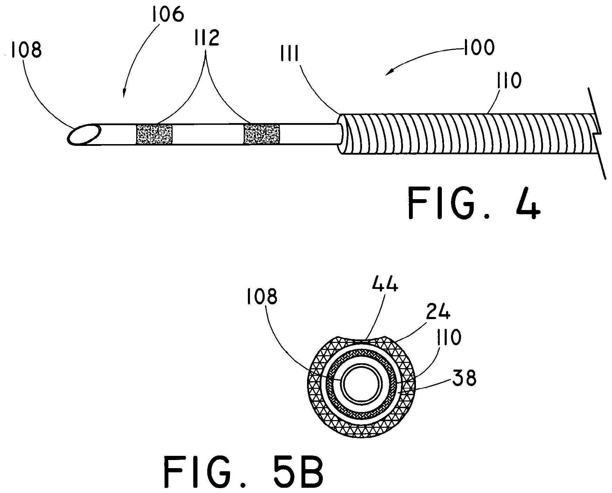

The second portion 24 may be connected to a handle 102 at a proximal end 104 of the medical device 100 as shown in FIG. 2. An exemplary distal end 106 of the medical device 100 is shown in FIG. 4 where the distal end 106 includes a sampling end, such as needle 108 that extends from a sheath 1 10 of the medical device 100. The needle may be made from any material known in the art, by way of non-limiting example, materials may include stainless steel or similar alloy, a memory-metal alloy, such as nickel titanium, a composite, a polymer, and/or a surgical stainless steel. The needle 100 may include an echogenic surface 112. Examples of echogenic markers on medical devices may be found in U.S. Published Application 200610247530. The needle 108 may be positioned within the sheath 1 10 as the sheath 1 10 of the medical device 100 is advanced through the channel 78 of the endoscope 20. The needle 108 and the sheath 110 are provided in a fixed rotational orientation in relation to the second portion 24 and are longitudinally movable in relation to the second portion 24 using the handle 102. The handle 102 may be used to releasably lock the needle 108 longitudinally in relation to the sheath 110. Advancement of the needle 108 at the biopsy site will be described below.

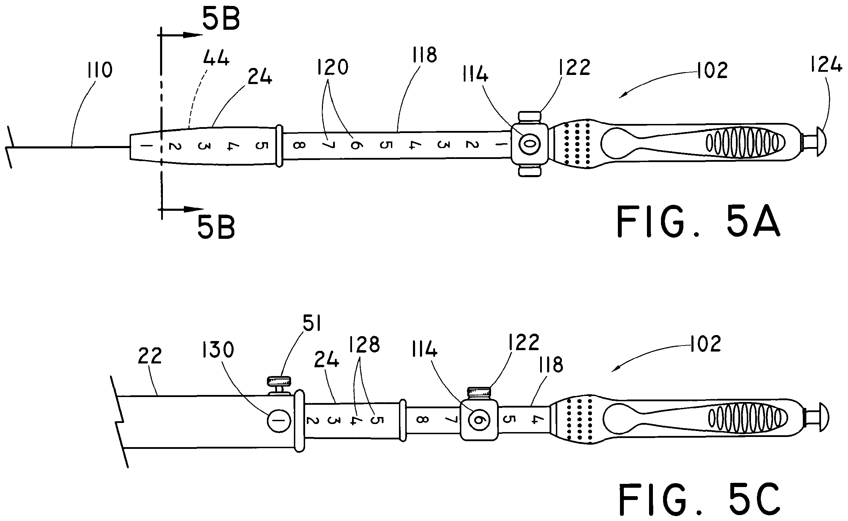

The exemplary handle 102 of the medical device 100 is shown in FIGS. 5A and 5C illustrating possible longitudinal changes in the position of the medical device 100 in relation to the second portion 24. FIG. 5B is a cross-sectional view of the second portion 24 taken across line 5B-5B of FIG. 5A. As shown in FIG. 5A, the handle 102 may include a needle adjuster 114 to adjust the length of the extension of the needle 108 out of the sheath 110. The needle adjuster may move along a shaft 118 of the handle 102 and include indicia 120 relating to the length that the needle 108 extends out of the sheath 110. A locking mechanism 122 may be used to releasably lock the needle adjuster in position. For example, the needle adjuster 114 positioned at 0 indicates that the needle 108 is positioned within the sheath 110 for insertion through the endoscope 20. A stylet 124 may extend longitudinally through the needle 108 to provide stiffness to the needle before sampling the tissue. FIG. 5B illustrates a cross sectional view of the second portion 24 having the keyway 44 and the sheath 110 and the needle 108 extending through the lumen 38 (stylet 124 not shown).

FIG. 5C illustrates the second portion 24 connected to the first portion 22 and rotationally secured. The second portion 24 may be longitudinally adjusted in relation to the second portion 22. Indicia 128 on the second portion 24 indicate the longitudinal position of the second portion 24 in relation to the first portion 22 and may be viewed through an opening 130 in the first portion 22. The locking mechanism 51 may be used to releasably secure the second portion 24. The needle adjuster 144 may be moved along the shaft 118 to extend the needle 108 out of the sheath 110 when the medical device has been fully inserted into the endoscope 20 and the distal end 76 of the endoscope 20 is in position within the patient for tissue sampling.

An exemplary endoscope is shown in FIG. 6. The endoscope 20 includes an operating control portion 162 in mechanical and fluid communication with an insertion tube 164. The operating control portion 162 is configured to control the insertion tube 164 and endoscopic parts disposed therein. As shown, the control portion 162 includes first and second control knobs 166, 168. The control knobs 166, 168 are configured to be in mechanical communication with the insertion tube 164. The control knobs 166, 168 allow the endoscopist to control and guide, by known means, the insertion tube 64 through the vessels and cavities of the patient. The control portion 162 may further include a plurality of ports, such as a suction port 170 and an air/water port 172. Each of the ports of the endoscope 20 is in communication to one of the working channels 173 of the insertion tube 164.

The endoscope 20 also includes an accessory channel 30 having a lumen 178 extending from the connector 28 to the distal end 176 of the endoscope 20. The accessory channel 30 is configured to receive medical devices, such as the medical device 100, therethrough for performing procedures through the distal end 176 of the endoscope 20 as is known in the art. The adaptor 10 described above is configured to removably connect to the connector 28 to orient the medical device 100 in relation to the endoscope 20.

As shown in FIG. 6, the exemplary endoscope 20 may further include an ultrasonic array of transducers 174 at the distal end 176 of the insertion tube 164. The transducers 174 may be connected to an imaging system (not shown) for viewing the image created by the ultrasonic transducers 174 and a medical device with an echogenic surface. The transducers 174 generate an ultrasonic scanning plane 180 to permit real-time monitoring of the medical device location and orientation within the scanning plane 180. Medical devices such as needles, including those for fine needle aspiration, wire guides, biopsy forceps and the like may include an echogenic surface and may be extended out of a port 182 in the distal end 176 of the endoscope 20 and viewed in the scanning plane 180.

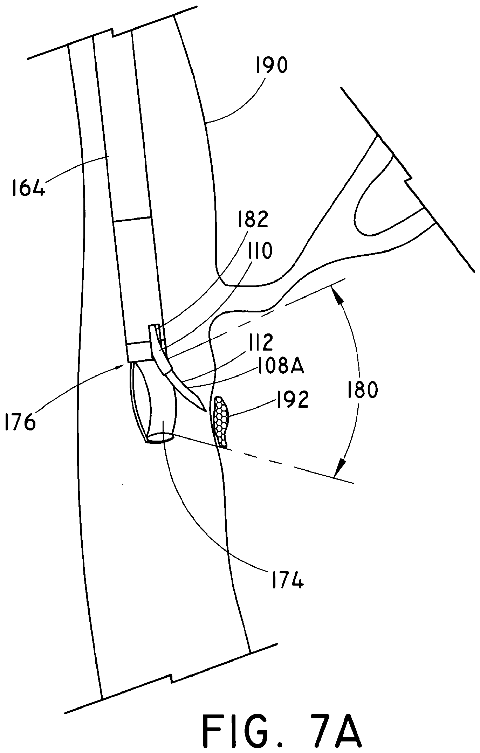

FIGS. 7A-7C illustrate the orientation of the exemplary medical device 100 using the adaptor 10 described above. By way of non-limiting example, the medical device 100 may be a needle, such as an EUS-FNA biopsy needle that may be used to sample lymph nodes, as well as masses arising in the lungs, pancreas, liver, adrenal gland and bile duct. In operation, the first portion 22 may be secured to the connector 28 of the endoscope 20 as shown in FIG. 2 to rotationally secure the first portion 22 in relation to the endoscope 20. A distal tip 111 if the sheath 110 of the medical device 100 (see FIG. 4) may be inserted into the lumen 32 of the first portion 22 and into the lumen 178 of the endoscope 20. The second portion 24 of the adaptor 10 is oriented so that the key 34 of the first portion and the keyway 44 of the second portion align, engage and are releasably connected. With the key 34 and the keyway 44 engaged, the second portion 24 is rotationally secured in relation to the first portion 22. The second portion 24 may be longitudinally moved in relation to the first portion 22 or the second portion 24 may also be secured longitudinally using for example, the thumb screw 51. The handle portion 102 may be longitudinally moved as describe above in relation to the second portion 24 and the endoscope 20 to extend the needle 108 and the sheath 110 of the medical device 100 out of the distal end portion of the endoscope 20.

FIG. 7A illustrates the distal end 176 of the insertion tube 164 of the endoscope 20 that has been advanced down through a bodily lumen 190 to a tissue mass 192 at a sampling site. The distal end 176 of the endoscope 20 is maneuvered as close as possible to the tissue mass 192. The sheath 110 is loaded into a proximal end 77 of the endoscope 20 through the accessory channel 30. In order to load and orient the needle 108, the adaptor 10 is used. As described above, the first portion 22 of the adaptor 10 is connected to the accessory channel 28 of the endoscope 20. The second portion 24 of the adaptor 10 is engaged and rotationally secured to the first portion 22. The second portion 24 is connected to the first portion 22 so that the key 34 receives the keyway 44 to rotationally secure the second portion 24 in relation to the second portion 22 of the adaptor 10. The needle 108 within the sheath 110 of the medical device 100 is longitudinally extended through the endoscope 20 and emerge from the port 182 at the distal end 176 of the endoscope 20.

The length of the adaptor 10 may be configured to be sufficient to allow the initial engagement, orientation and rotational securing of the second portion 24 in relation to the first portion 22 before the medical device 100 extends out of the distal end 176 of the endoscope 20. Orienting and rotationally securing the second portion 24 in relation to the first portion 22 using the key 34/keyway 44 before the medical device 100 extends out of the distal end 176 of the endoscope 20 allows the medical device 100 to be oriented before the medical device 100 passes through the portion of the endoscope 20 that induces bending into the medical device 100 as described above. The bend-inducing portion of the endoscope may be at the port 182 of the distal end 176, by way of non-limiting example. However, one skilled in the art will understand that the bend-inducing portion may also be at other positions along the working channel 173 of the endoscope 20.

The relationship between the length of the engagement region where the key 34 and the keyway 44 of the adaptor 10 initially connect and the distal extension of the medical device 100 is illustrated in the following example. If the length of the elongate medical device 100 that extends beyond the bend-inducing portion of the endoscope 20 is about 10 cm, then the length of the engagement region between the key 34 and the keyway 44 is at least about 10 cm so that the orientation of the medical device 100 relative to the endoscope 20 begins with the engagement of the key 34 and the keyway 44 and before the medical device 100 extends through the bend inducing portion.

FIG. 7A illustrates the needle 108 in the first position 108a for the first extension of the needle 108 into the scanning plane 180 generated by the ultrasonic transducers 174. In the first position 108a, reflections off an echogenic surface 112 on the needle 108 allow visualization of the path of the needle 108 relative to the mass 192 to be monitored. After the needle 108 has been guided to the tissue mass 192, the endoscopist may puncture the mass with swift back and forth movements of the needle 108. Upon successful sampling of the tissue mass 192, the needle 108 may be longitudinally retracted out of the scanning plane 180 and the second portion 24 of the adaptor 10 disconnected from the first portion 22 and the medical device 100 removed from the endoscope. Aspiration of the contents from the tissue mass 192 includes may include application of negative pressure with a syringe (not shown) over the sheath 110. As the needle 108 is retracted longitudinally through the port 182 to and out of the accessory channel, the needle may become bent or curved.

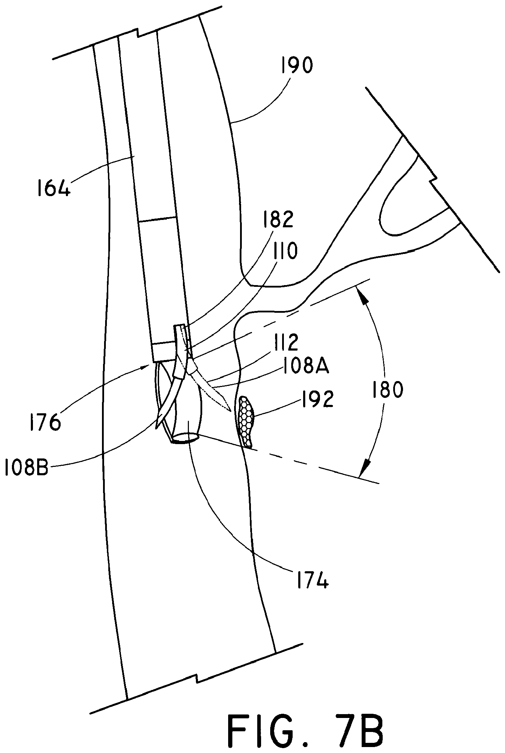

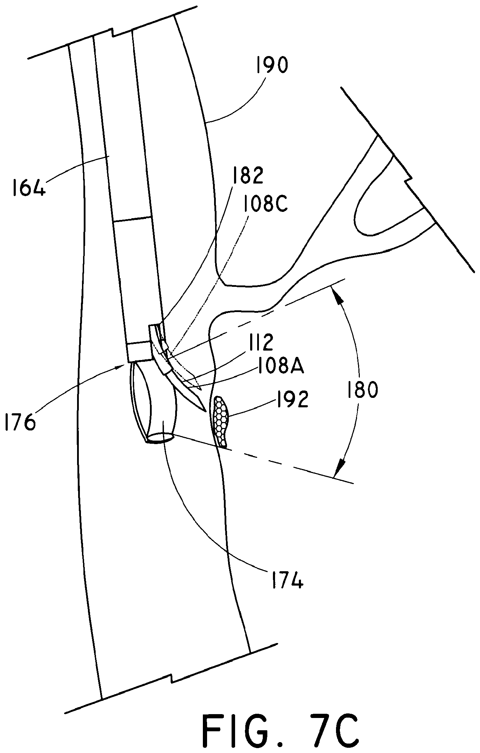

One or more additional samplings may be completed while the distal end 176 of the endoscope 20 remains optimally positioned near the tissue mass 192. The second portion 24 of the adaptor 10 may be reconnected to the first portion 22 in the same rotational orientation as the original connection using the key/keyway 34, 44 orientation to ensure that the needle 108 will extend in substantially the same position 108a as in the first sampling procedure shown in FIG. 7A. The rotational orientation of the needle 108 using the adaptor 10 is shown for the first extension in FIG. 7A and subsequent extensions in FIG. 7C. If the adaptor 10 is not used to rotationally orient the sheath 110 and needle 108, the needle 108 may extend from the port 182 of the distal end 176 of the endoscope 20 in an angle outside of the scanning plane 180 as shown in FIG. 7B. FIG. 7B illustrates a curved needle position 108b that extends out of the scanning plane 180 so that the needle 108 cannot be visualized and a tissue sample cannot be take at the tissue mass 192.

In an alternative operation, the second portion 24 may be connected to the first portion 22 by orienting and engaging the key 34 and the keyway 44 and rotationally securing the second portion 24 in relation to the first portion 22 before connecting the first portion 22 to the endoscope 20. The first portion 22 may then be connected to the connector 28 on the accessory channel 30 of the endoscope 20 having the elongate medical device 100 inserted into the endoscope 20 before completing the connection of the first portion 22 to the connector 28. Once the first portion 22 is connected to the connector 28, the first portion 22 remains connected and rotationally secured in relation to the endoscope 20 through out the procedure. A sample may be taken and the second portion 24 may be released from the first portion 22 to remove the medical device 100 from the endoscope 20. The medical device 100 may be reinserted into the endoscope 20 as described above and the second portion 24 reconnected to the first portion 22 so that the medical device 100 is oriented with respect to the endoscope 20 in the same rotational relationship as the first sampling relationship and the needle 108 extends into the viewing plane 180 as described above.

As will be understood by a skilled artisan, the orienting adaptor may also be used with a conventional image system also having a viewing plane where a curved medical device may be re-extended from the distal tip of the endoscope outside of the viewing plane. In addition, many types of elongate medical devices, used for multiple extensions through the endoscope distal end and having an operational direction for the patient procedure may be used with the adaptor of the present invention.

The above Figures and disclosure are intended to be illustrative and not exhaustive. This description will suggest many variations and alternatives to one of ordinary skill in the art. All such variations and alternatives are intended to be encompassed within the scope of the attached claims. Those familiar with the art may recognize other equivalents to the specific embodiments described herein which equivalents are also intended to be encompassed by the attached claims. For example, the invention has been described using an EUS needle for illustrative purposes only. Application of the principles of the invention to any other elongate medical device are within the ordinary skill in the art and are intended to be encompassed within the scope of the attached claims.

## Claims

1. The assembly of an elongate medical device and an adaptor to orient the elongate medical device in relation to an endoscope, the adaptor (10) comprising:
a first portion (22) having a distal end connectable and rotationally securable to the endoscope and a proximal end, the first portion having a first lumen defined longitudinally therethrough and one of an orienting key (34) or a keyway (44) extending longitudinally at least partially along the first portion;
a second portion (24) connectable to the first portion and including a second lumen extending longitudinally through the second portion and operably connectable to the first lumen, the second portion having the other of the key (34) or the keyway (44) extending longitudinally at least partially along the second portion, the keyway configured to releasably mate with the key to orient and rotationally secure the second portion relative to the first portion; and the elongate medical device (100) comprising:
a handle (102) connectable to the second portion, the handle configured to adjust the longitudinal position of the medical device relative to the second portion; and
a needle (108) extendable distally through the endoscope, the needle comprising an echogenic surface and a tip that is orientable in relation to a distal portion of the endoscope;
wherein the second portion is configured to receive the elongate medical device longitudinally movable in relation to the second portion and rotationally secured relative to the second portion; wherein the adaptor is configured to orient said needle tip relative to the endoscope, so that said needle tip is extendible from the distal portion of the endoscope in the same orientation for the first and subsequent extensions of the needle from the endoscope.

2. The assembly of claim 1, further comprising an endoscope, wherein the first portion of the adaptor is connected and rotationally secured to the endoscope.

3. The assembly of claim 2, wherein the endoscope further comprises at least one transducer to emit ultrasound waves.

4. The assembly of claim 1, wherein the first portion comprises the key and the second portion comprises the keyway.

5. The assembly of claim 1, wherein the first portion further comprises a releasable lock to secure the longitudinal position of the second portion relative to the first portion.

6. The assembly of claim 1, wherein the first portion is configured for releasable connection to the endoscope.

## Patentansprüche

1. Anordnung einer länglichen medizinischen Vorrichtung und Adapter zur Ausrichtung der länglichen medizinischen Vorrichtung bezüglich eines Endoskops, wobei der Adapter (10) Folgendes umfasst:
einen ersten Abschnitt (22) mit einem distalen Ende, das mit dem Endoskop verbunden und drehungsmäßig daran befestigt werden kann, und einem proximalen Ende, wobei der erste Abschnitt ein erstes Lumen, das in Längsrichtung dort hindurch definiert ist, und einen Ausrichtungskeil (34) oder eine Keilnut (44) hat, der bzw. die sich in Längsrichtung mindestens teilweise am ersten Abschnitt entlang erstreckt;
einen zweiten Abschnitt (24), der mit dem ersten Abschnitt verbindbar ist und ein zweites Lumen aufweist, das sich in Längsrichtung durch den zweiten Abschnitt erstreckt und mit dem ersten Lumen wirkverbindbar ist, wobei der zweite Abschnitt den bzw. die jeweils andere(n) des Keils (34) oder der Keilnut (44) hat, der bzw. die sich in Längsrichtung mindestens teilweise am zweiten Abschnitt entlang erstreckt, wobei die Keilnut zum freigebbaren Eingriff mit dem Keil konfiguriert ist, um den zweiten Abschnitt bezüglich des ersten Abschnitts auszurichten und drehungsmäßig zu befestigen; und
wobei die längliche medizinische Vorrichtung (100) Folgendes umfasst:
einen mit dem zweiten Abschnitt verbindbaren Griff (102), wobei der Griff dazu konfiguriert ist, die Längsposition der medizinischen Vorrichtung bezüglich des zweiten Abschnitts zu verstellen; und
eine distal durch das Endoskop ausfahrbare Nadel (108), wobei die Nadel eine echogene Oberfläche und eine Spitze umfasst, die bezüglich eines distalen Abschnitts des Endoskops ausrichtbar ist;
wobei der zweite Abschnitt dazu konfiguriert ist, die längliche medizinische Vorrichtung in Längsrichtung bezüglich des zweiten Abschnitts beweglich und drehungsmäßig bezüglich des zweiten Abschnitts befestigt aufzunehmen;
wobei der Adapter dazu konfiguriert ist, die Nadelspitze bezüglich des Endoskops auszurichten, so dass die Nadelspitze für das erste und für nachfolgendes Ausfahren der Nadel aus dem Endoskop in derselben Ausrichtung aus dem distalen Abschnitt des Endoskops ausfahrbar ist.

2. Anordnung nach Anspruch 1, ferner umfassend ein Endoskop, wobei der erste Abschnitt des Adapters mit dem Endoskop verbunden und drehungsmäßig daran befestigt ist.

3. Anordnung nach Anspruch 2, wobei das Endoskop ferner mindestens einen Wandler zum Emittieren von Ultraschallwellen umfasst.

4. Anordnung nach Anspruch 1, wobei der erste Abschnitt den Keil und der zweite Abschnitt die Keilnut umfasst.

5. Anordnung nach Anspruch 1, wobei der erste Abschnitt ferner eine freigebbare Verriegelung zur Befestigung der Längsposition des zweiten Abschnitts bezüglich des ersten Abschnitts umfasst.

6. Anordnung nach Anspruch 1, wobei der erste Abschnitt zur freigebbaren Verbindung mit dem Endoskop konfiguriert ist.

## Revendications

1. Ensemble composé d'un dispositif médical allongé et d'un adaptateur servant à orienter le dispositif médical allongé par rapport à un endoscope, l'adaptateur (10) comprenant :
une première partie (22) comportant une extrémité distale pouvant être raccordée et solidarisée en rotation avec l'endoscope et une extrémité proximale, la première partie comportant une première lumière définie longitudinalement à travers elle et un élément parmi une clavette d'orientation (34) et une rainure de clavette (44) s'étendant longitudinalement au moins partiellement le long de la première partie ;
une seconde partie (24) pouvant être raccordée à la première partie et comprenant une seconde lumière s'étendant longitudinalement à travers la seconde partie et pouvant être raccordée de manière fonctionnelle à la première lumière, la seconde partie comportant l'autre élément parmi la clavette (34) et la rainure de clavette (44) s'étendant longitudinalement au moins partiellement le long de la seconde partie, la rainure de clavette étant configurée pour s'accoupler de manière libérable avec la clavette afin d'orienter et de solidariser en rotation la seconde partie vis-à-vis de la première partie ; et
le dispositif médical allongé (100) comprenant :
un manche (102) pouvant être raccordé à la seconde partie, le manche étant configuré pour ajuster la position longitudinale du dispositif médical par rapport à la seconde partie ; et
une aiguille (108) extensible dans la direction distale à travers l'endoscope, l'aiguille comprenant une surface échogène et une pointe qui est orientable par rapport à une partie distale de l'endoscope ;
la seconde partie étant configurée pour recevoir le dispositif médical allongé de sorte qu'il soit mobile longitudinalement vis-à-vis de la seconde partie et solidarisé en rotation vis-à-vis de la seconde partie ;
l'adaptateur étant configuré pour orienter ladite pointe d'aiguille par rapport à l'endoscope, de telle sorte que ladite pointe d'aiguille soit extensible à partir de la partie distale de l'endoscope dans la même orientation pour la première extension et les extensions suivantes de l'aiguille à partir de l'endoscope.

2. Ensemble selon la revendication 1, comprenant en outre un endoscope, dans lequel la première partie de l'adaptateur est raccordée et solidarisée en rotation avec l'endoscope.

3. Ensemble selon la revendication 2, dans lequel l'endoscope comprend en outre au moins un transducteur servant à émettre des ondes ultrasonores.

4. Ensemble selon la revendication 1, dans lequel la première partie comprend la clavette et la seconde partie comprend la rainure de clavette.

5. Ensemble selon la revendication 1, dans lequel la première partie comprend en outre un élément de blocage libérable servant à fixer la position longitudinale de la seconde partie par rapport à la première partie.

6. Ensemble selon la revendication 1, dans lequel la première partie est configurée à des fins de raccordement libérable avec l'endoscope.
